# EUROPEAN PATENT APPLICATION

(11) **EP 4 310 173 A1**
(43) Date of publication of application: **24.01.2024**
(21) Application number: 22791578.2
(22) Date of filing: 31.03.2022
(51) Int. Cl.: C12M 1/42, C12N 5/10, C12N 1/15, C12N 1/19, C12N 1/21, C12N 15/87

(54) **ELECTRIC PUNCHING DEVICE, INTRODUCTION METHOD OF BIOACTIVE MATERIAL, PRODUCING METHOD OF TISSUE-DERIVED BIOMATERIAL, AND TISSUE-DERIVED BIOMATERIAL**

(30) Priority: 22.04.2021 JP 2021072675
(71) Applicant: FUJIFILM Corporation, Tokyo 106-8620 (JP)
(72) Inventor: KOBAYASHI, Yuka, Ashigarakami-gun, Kanagawa 258-8577 (JP); YAKUSHIJI, Takashi, Ashigarakami-gun, Kanagawa 258-8577 (JP)
(74) Representative: Hoffmann Eitle
(86) International application number: PCT/JP2022/016717
(87) International publication number: WO 2022/224803

(57) **Abstract**

Provided is an electric perforating device 10 including a flow passage 20 through which liquid flows, a first electrode 30A that forms a first electric field region 60A in the flow passage 20 by being supplied with a pulse voltage, a second electrode 30B that forms a second electric field region 60B on a downstream side of the first electric field region 60A, which is in the flow passage 20, in a flowing direction of the liquid, by being supplied with a pulse voltage, a cooling portion 40 that forms a cooling region 70, which cools the liquid flowing through the flow passage 20, between the first electric field region 60A and the second electric field region 60B in the flow passage 20, and a voltage output portion 50 that outputs a pulse voltage for forming the first electric field region 60A and the second electric field region 60B.

## Description

### BACKGROUND OF THE INVENTION

### 1. Field of the Invention

The disclosed technology relates to an electric perforating device, an introduction method of a bioactive substance, a manufacturing method of a biologically derived material, and a biologically derived material.

### 2. Description of the Related Art

An electric perforating method is a method of introducing a substance into a cell by perforating a hole in a cell membrane with an electric pulse and is also called electroporation. For example, a fine hole can be made in the cell membrane by applying an electric pulse to cell suspension, and a cell can be transformed by introducing deoxyribonucleic acid (DNA) into the cell. The following technology is known as a technology for introducing a bioactive substance such as DNA into a cell by using an electric perforating method.

For example, JP2007-7430A discloses an electroporation device including: a fluid flow passage; an electrode to which biological particles, which are disposed along the fluid flow passage and moved along the fluid flow passage, are attached to an electric field suitable for electroporation; and a computer that controls a pulse charge applied to the electrode, in which a method of cooling the electrode is included.

JP2011-528550A discloses an electric perforating method including: a step of determining a first time constant (t1) representing an increase in conductivity in an electric perforation culture medium in a case where a pulse is applied; and a step of determining a second time constant (t2) representing a discharge of a capacitor, in which an electric perforation parameter is set such that t1 is greater than or equal to t2 and a duration period of the pulse is shorter than either t1 or t2, and one or a plurality of electric pulses are applied to a sample where an electric perforation is performed by using this electric perforation parameter.

US2017/0283761A describes a device for electroporation of cells including two electrodes.

### SUMMARY OF THE INVENTION

It is known that there is a flow process of causing suspension, which contains a biologically derived material and a bioactive substance, to flow in a flow passage, where a parallel flat plate electrode is disposed, as a process of introducing a bioactive substance such as DNA, ribonucleic acid (RNA), and proteins into a biologically derived material such as a cell, a cell derivative, a cell organelle, an intracellular granule, and a vesicle by using an electric perforating method.

The following points are mentioned as problems in the flow process. The Joule heat generated in a case where a voltage is applied heats the suspension flowing through the flow passage, and this heat may damage the biologically derived material. Further, there is a concern that an explosive boil occurs in the heated suspension, and a shock wave generated due to the explosive boil damages the biologically derived material. Further, in a case where the temperature of the electrode is excessively high, the deterioration of the electrode progresses, and the life of the electric perforating device is shortened.

In the electric perforation using the flow process, as a result of careful examination of the temperature rise of the suspension flowing through the flow passage, the present inventors have found that the electrode and the suspension become hot locally and instantaneously even under a condition where the temperature of the electrode and the suspension measured by temporally and spatially averaged is sufficiently suppressed. That is, it is considered that the above-described problems are solved by suppressing local and instantaneous temperature rises of the electrode and the suspension.

The object of the disclosed technology is to suppress local and instantaneous temperature rises of an electrode and suspension in electric perforation using a flow process.

An electric perforating device according to the disclosed technology includes: a flow passage through which liquid flows; a first electrode that forms a first electric field region in the flow passage by being supplied with a pulse voltage; a second electrode that forms a second electric field region on a downstream side of the first electric field region, which is in the flow passage, in a flowing direction of the liquid, by being supplied with a pulse voltage; a cooling portion that forms a cooling region, which cools the liquid flowing through the flow passage, between the first electric field region and the second electric field region in the flow passage; and a voltage output portion that outputs a pulse voltage for forming the first electric field region and the second electric field region.

The electric perforating device may further include a cooling mechanism that cools the first electrode and the second electrode.

The electric perforating device may include a plurality of pairs of conductive members provided on wall surfaces of the flow passage facing each other along the flowing direction of the liquid and a cooling mechanism that cools each of the plurality of pairs of conductive members. In this case, by supplying a pulse voltage with the voltage output portion to each of two pairs of conductive members between which at least one pair of conductive members, among the plurality of pairs of conductive members, is interposed, one of the two pairs of conductive members may function as the first electrode, the other of the two pairs of conductive members may function as the second electrode, and the at least one pair of conductive members, which is interposed between the two pairs of conductive members, may function as the cooling portion. The voltage output portion may switch the conductive member, to which the pulse voltage is supplied among the plurality of pairs of conductive members, at a predetermined timing. The plurality of pairs of conductive members may have the same length in the flowing direction of the liquid.

An introduction method of a bioactive substance according to the disclosed technology is an introduction method of introducing a bioactive substance into a biologically derived material while suspension containing the biologically derived material and the bioactive substance flows through a flow passage, the introduction method includes: a step of causing the suspension to pass through a first electric field region; a step of causing the suspension to pass through a cooling region, which cools the suspension, after the suspension has passed through the first electric field region; and a step of causing the suspension to pass through a second electric field region after the suspension has passed through the cooling region.

A step of causing the suspension to pass through an electric field region and the step of causing the suspension to pass through the cooling region may be alternately repeated two or more times each.

An accumulated amount of energy, which is applied to the suspension while the suspension passes through a plurality of electric field regions including the first electric field region and the second electric field region, may correspond to an amount of energy predetermined as energy required for introducing the bioactive substance into the biologically derived material.

A local and instantaneous maximum temperature of the suspension in the flow passage is preferably 100°C or lower.

A plurality of electric field regions including the first electric field region and the second electric field region may be each formed by supplying a pulse voltage to a cooled electrode.

Time required for the suspension to pass through one electric field region is preferably an integer multiple of a period of the pulse voltage.

The biologically derived material is not limited as long as it is a cell and may be a human derived cell. Particularly preferably, the biologically derived material is a HEK 293 cell. The bioactive substance may be DNA.

A manufacturing method of a biologically derived material according to the disclosed technology includes a step of introducing a bioactive substance by using the introduction method.

A biologically derived material according to the disclosed technology is a biologically derived material that is manufactured by using the manufacturing method.

According to the disclosed technology, it is possible to suppress local and instantaneous temperature rises of an electrode and suspension in electric perforation using a flow process.

### BRIEF DESCRIPTION OF THE DRAWINGS

Fig. 1 is a perspective view schematically showing a configuration of an electric perforating device according to an embodiment of the disclosed technology.
Fig. 2 is a cross-sectional view taken along the line 2-2 in Fig. 1.
Fig. 3 is a diagram showing an example of a pulse voltage output from a voltage output portion according to the embodiment of the disclosed technology.
Fig. 4 is a cross-sectional view showing an example of a configuration of a cooling portion according to the embodiment of the disclosed technology.
Fig. 5 is a perspective view showing a configuration of an electric perforating device according to Comparative Example.
Fig. 6 is an image showing an aspect in a case where a pulse voltage is applied to an electrode while causing suspension to flow in a flow passage in the electric perforating device according to Comparative Example.
Fig. 7 is an image showing an aspect in a case where a pulse voltage is applied to the electrode while causing suspension to flow in the flow passage in the electric perforating device according to Comparative Example.
Fig. 8 is a cross-sectional view showing a configuration of an electric perforating device according to another embodiment of the disclosed technology.
Fig. 9 is a diagram showing a simulation model of an electric perforating device according to Example of the disclosed technology.
Fig. 10 is a diagram showing a simulation model of the electric perforating device according to Comparative Example.
Fig. 11A is a contour diagram showing a temperature distribution of fluid in a model according to Example of the disclosed technology.
Fig. 11B is a contour diagram showing a temperature distribution of fluid in a model according to Example of the disclosed technology.
Fig. 12A is a contour diagram showing a temperature distribution of fluid in a model according to Comparative Example.
Fig. 12B is a contour diagram showing a temperature distribution of fluid in a model according to Comparative Example.
Fig. 13 is a graph showing a time transition of the temperature of fluid in a model according to Example of the disclosed technology.
Fig. 14 is a graph showing a time transition of the temperature of fluid in a model according to Example of the disclosed technology.
Fig. 15 is a cross-sectional view showing an example of a configuration of an electric perforating device according to still another embodiment of the disclosed technology.
Fig. 16 is a cross-sectional view showing an example of a configuration of an electric perforating device according to still another embodiment of the disclosed technology.
Fig. 17 is a cross-sectional view showing an example of a usage form of an electric perforating device according to still another embodiment of the disclosed technology.
Fig. 18 is a cross-sectional view showing an example of a usage form of an electric perforating device according to still another embodiment of the disclosed technology.

### DESCRIPTION OF THE PREFERRED EMBODIMENTS

Hereinafter, an example of an embodiment of the technology of the present disclosure will be described with reference to the drawings. It should be noted that the same or equivalent components and portions in the drawings are assigned by the same reference numerals, and the overlapping description will be omitted.

In the present specification, a biologically derived material is a structure that is derived from a living body such as a cell, a cell derivative, a cell organelle, an intracellular granule, and a vesicle. The biologically derived material may include yeast, coliform bacteria, or animal cells, preferably animal cells, and the animal cells may be human derived cells. Human T cells, HEK293, A549, SF9, EB66, Daudi, Hela, Vero, and MDCK may be used, or HEK293 cells may be used. The bioactive substance may be any substance that exerts some action on the biologically derived material and may be DNA, RNA, or protein. The bioactive substance includes, for example, a plasmid, linear DNA, mRNA, and the like.

### [First Embodiment]

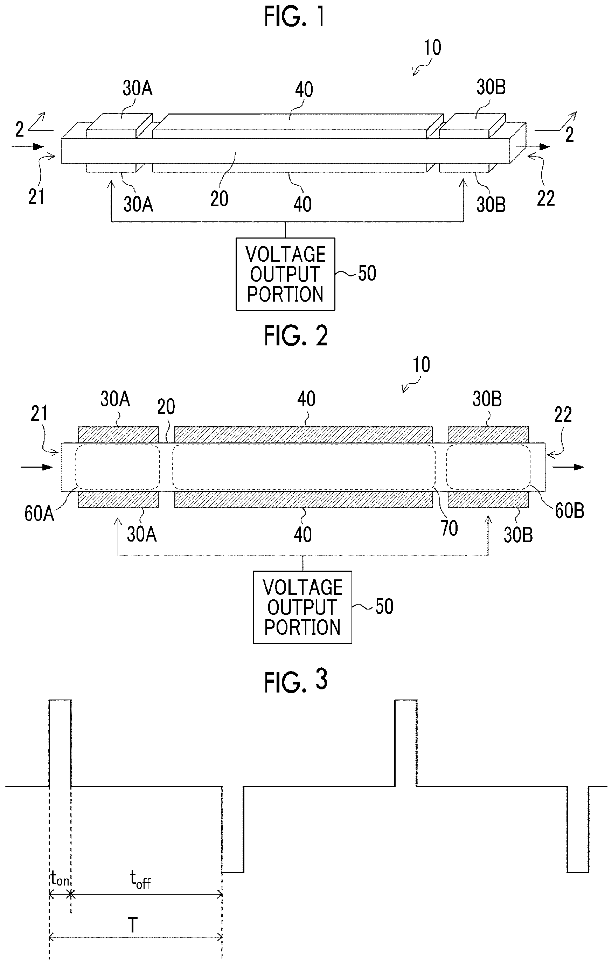

Fig. 1 is a perspective view schematically showing a configuration of an electric perforating device 10 according to a first embodiment of the disclosed technology. Fig. 2 is a cross-sectional view taken along the line 2-2 in Fig. 1. The electric perforating device 10 is a device used for introducing a bioactive substance into a biologically derived material by using an electric perforating method using a flow process. For example, by introducing DNA, which is an example of a bioactive substance, into a cell, which is an example of a biologically derived material, by using the electric perforating device 10, it is possible to create a new cell having new genetic characteristics. The electric perforating device 10 includes a flow passage 20, a first electrode 30A, a second electrode 30B, a cooling portion 40, and a voltage output portion 50.

The flow passage 20 is a flow passage where liquid (hereinafter, referred to as suspension), which contains a biologically derived material and a bioactive substance, flows. The flow passage 20 includes an inlet 21 and an outlet 22. The inlet 21 is provided on one end side of the flow passage 20, and the outlet 22 is provided on the other end side of the flow passage 20. The flow passage 20 is composed of an insulator such as resin or glass. In the case of using the electric perforating device 10, the suspension flows into the flow passage 20 through the inlet 21, flows through the flow passage 20, and flows out of the flow passage 20 through the outlet 22. In the present embodiment, an area of the cross section of the flow passage 20 orthogonal to a flowing direction of the suspension is set to be constant. Further, a flow velocity of the suspension flowing through the flow passage 20 is set to be constant. A liquid flow of the suspension flowing through the flow passage 20 is formed by a liquid feeding unit such as a pump (not shown).

The first electrode 30A has a form of a so-called parallel flat plate electrode and is configured to include a pair of conductive members provided on wall surfaces of the flow passage 20 facing each other. The first electrode 30A forms a first electric field region 60A in the flow passage 20 by being supplied with a pulse voltage output from the voltage output portion 50.

Similar to the first electrode 30A, the second electrode 30B has a form of a parallel flat plate electrode and is configured to include a pair of conductive members provided on the wall surfaces of the flow passage 20 facing each other. The second electrode 30B is provided on a downstream side of the first electrode 30A in the flowing direction of the suspension flowing through the flow passage 20. The second electrode 30B forms a second electric field region 60B on the downstream side of the first electric field region 60A, which is in the flow passage 20, in the flowing direction of the suspension flowing through the flow passage 20 by being supplied with the pulse voltage output from the voltage output portion 50. Dimensions (a length in the flowing direction of the suspension) of the first electrode 30A and the second electrode 30B are preferably the same. As the conductive member constituting the first electrode 30A and the second electrode 30B, for example, a metal such as Cu or Al or a conductive resin can be used.

The voltage output portion 50 supplies the pulse voltage to each of the first electrode 30A and the second electrode 30B. Fig. 3 is a diagram showing an example of the pulse voltage output from the voltage output portion 50. The voltage output portion 50 outputs the pulse voltage of which polarities are alternately switched. A period T of the pulse voltage is a sum of an ON period tₒₙ and an OFF period t_{off}. Time required for the suspension to pass through one electric field region (a region directly under the electrode) of the first electric field region 60A and the second electric field region 60B can be an integer multiple of the period T of the pulse voltage. Accordingly, voltage application time for the biologically derived material contained in the suspension flowing through the flow passage 20 becomes uniform regardless of the time at which the pulse voltage is ON. For example, in a case where the time required for the suspension to pass through one electric field region is one times the period T of the pulse voltage, the voltage application time for the biologically derived material within a period when the biologically derived material passes through one electric field region (the region directly under the electrode) is equal to the ON period tₒₙ regardless of the time at which the pulse voltage is ON. Although the suspension flowing through the flow passage 20 is considered to have a flow velocity distribution, in calculating the time required for the suspension to pass through the electric field region, it is possible to use a representative value such as the maximum value and an average value.

In the electric perforating device 10 according to the present embodiment, it is considered that the amount of energy, which is predetermined as the amount of energy E_{N} required for introducing the bioactive substance into the biologically derived material, is divided and applied to the first electric field region 60A and the second electric field region 60B. That is, the ON period tₒₙ and a voltage level of the voltage pulse are set such that an accumulated amount of energy E_{G}, which is applied to the suspension while the suspension passes through the first electric field region 60A and the second electric field region 60B, corresponds to a required amount of energy E_{N} (that is, so that E_{G} ≈ E_{N}). By making the dimensions of the first electrode 30A and the second electrode 30B the same (the length in the flowing direction of the suspension), the required amount of energy E_{N} can be divided evenly and applied to each electric field region.

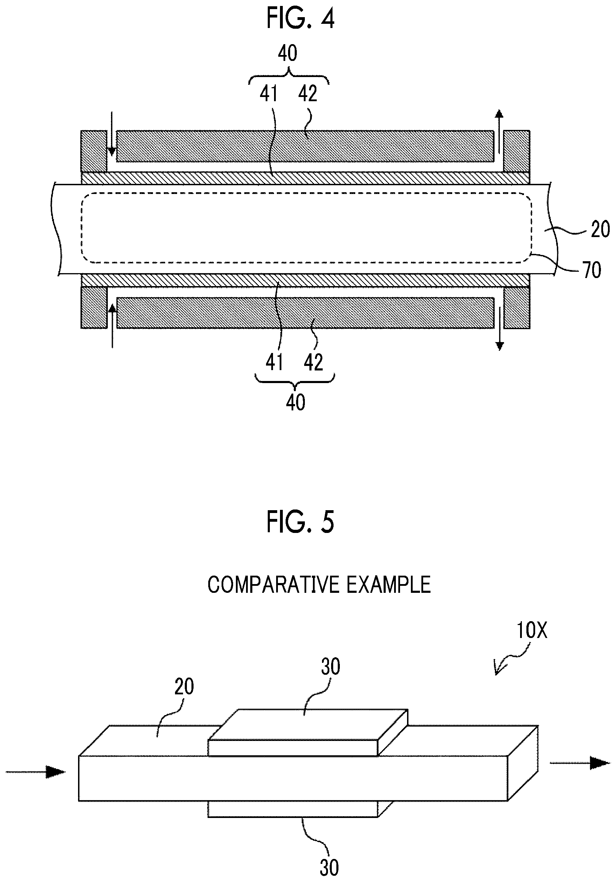

The cooling portion 40 is provided between the first electrode 30A and the second electrode 30B of the flow passage 20. The cooling portion 40 forms a cooling region 70, which cools the suspension flowing through the flow passage 20, between the first electric field region 60A and the second electric field region 60B in the flow passage 20. Fig. 4 is a cross-sectional view showing an example of a configuration of the cooling portion 40. The cooling portions 40 are configured to include heat conduction members 41 provided on wall surfaces of the flow passage 20 facing each other and cooling mechanisms 42 connected to the heat conduction members 41. The heat conduction member 41 is preferably made of, for example, a metal having relatively high thermal conductivity, such as Cu and Al. The cooling mechanism 42 may be, for example, a water cooling type mechanism through which liquid as a cooling medium flows inside a jacket, as illustrated in Fig. 4. From the viewpoint of cooling efficiency, it is preferable that coolant comes into direct contact with the heat conduction member 41. Further, it is also possible to integrally configure the heat conduction member 41 and the cooling mechanism 42 by providing a flow passage through which the cooling medium flows inside the heat conduction member 41. Further, the cooling mechanism 42 is not limited to a water cooling type mechanism and may be, for example, an air cooling type mechanism or a mechanism using a Peltier element.

The suspension is continuously cooled while the suspension passes through the cooling region 70. Therefore, the longer the length of the cooling portion 40 (the cooling region 70) in the flowing direction of the suspension, the lower the temperature of the suspension can be. In the electric perforating device 10, a length of the first electrode 30A (the first electric field region 60A), a length of the second electrode 30B (the second electric field region 60B), and a length of the cooling portion 40 (the cooling region 70) in the flowing direction of the suspension are set such that the local and instantaneous maximum temperature of the suspension in the flow passage 20 is 100°C or lower. That is, the length of the cooling portion 40 is set such that the temperature of the suspension can be reduced before the suspension reaches the second electric field region to a temperature of 100°C or lower even in a case where the suspension is heated in the second electric field region 60B.

According to the electric perforating device 10 of the present embodiment, an introduction method of introducing the bioactive substance into the biologically derived material while the suspension containing the biologically derived material and the bioactive substance flows through the flow passage 20, is implemented. That is, the introduction method according to the embodiment of the disclosed technology includes a step of causing the suspension to pass through the first electric field region 60A, a step of causing the suspension to pass through the cooling region 70, which cools the suspension, after the suspension has passed through the first electric field region, and a step of causing the suspension to pass through the second electric field region 60B after the suspension has passed through the cooling region 70.

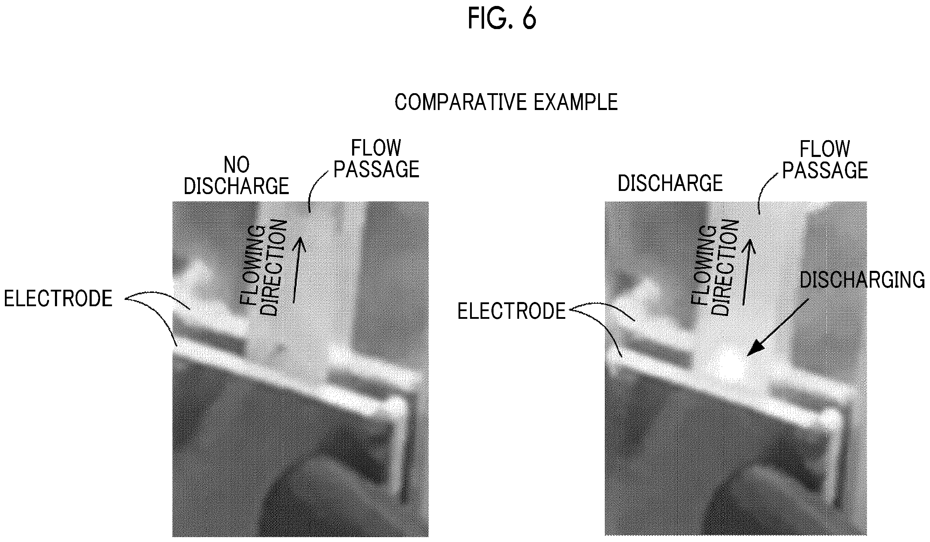

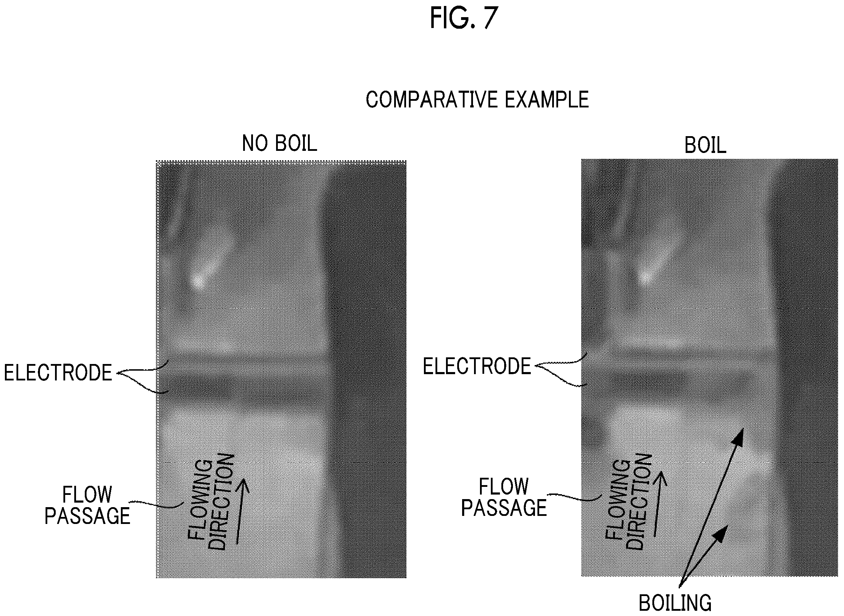

Fig. 5 is a perspective view showing a configuration of an electric perforating device 10X according to Comparative Example which does not have a cooling portion. The electric perforating device 10X according to Comparative Example includes a flow passage 20 and a single electrode 30. Figs. 6 and 7 are images showing an aspect in a case where a pulse voltage is applied to the electrode 30 while causing the suspension to flow in the flow passage 20 in the electric perforating device 10X according to Comparative Example.

As shown in an image on the right side in Fig. 6 and an image on the right side in Fig. 7, according to the electric perforating device 10X of Comparative Example, the suspension flowing through the flow passage 20 is heated due to Joule heat generated in a case where a voltage is applied, and then the explosive boil of the suspension (see the right side in Fig. 7) and a discharge phenomenon at an end portion on an electrode outlet side (see the right side in Fig. 6) have occurred. According to the electric perforating device 10X according to Comparative Example, there is a possibility that the biologically derived material contained in the suspension is damaged due to heat and shock waves associated with explosive boil or discharge. Further, there is a possibility that deterioration of the electrode 30 progresses, and the life of the electric perforating device 10X is shortened.

On the other hand, according to the electric perforating device 10 of the embodiment of the disclosed technology, since the first electrode 30A and the second electrode 30B, which are separated from each other, are provided, the amount of energy E_{N} required for introducing the bioactive substance into the biologically derived material can be divided and applied to the first electric field region 60A and the second electric field region 60B. Accordingly, it is possible to suppress the amount of energy in one electric field region, and thus it is possible to suppress local and instantaneous temperature rises of the electrode and the suspension.

Here, as a method of suppressing local and instantaneous temperature rises of the electrode and the suspension, it is conceivable to lengthen the period T and OFF period t_{off} of the pulse voltage. However, in this case, it is necessary to reduce the flow velocity of the suspension to ensure the amount of energy E_{N} required for introducing the bioactive substance into the biologically derived material, and then the processing efficiency is reduced. According to the electric perforating device 10 according to the embodiment of the disclosed technology, it is possible to provide a required amount of energy E_{N} without reducing the flow velocity of the suspension. That is, according to the electric perforating device 10 according to the embodiment of the disclosed technology, in the electric perforation using the flow process, it is possible to suppress local and instantaneous temperature rises of the electrode and the suspension without reducing the processing efficiency (throughput).

Further, according to the electric perforating device 10 of the embodiment of the disclosed technology, since the cooling portion 40 is provided between the first electrode 30A and the second electrode 30B, the suspension, of which the temperature has risen by passing through the first electric field region 60A, is cooled before passing through the second electric field region 60B. Accordingly, it is possible to suppress the local and instantaneous maximum temperature determined in response to the cumulative temperature change of the suspension.

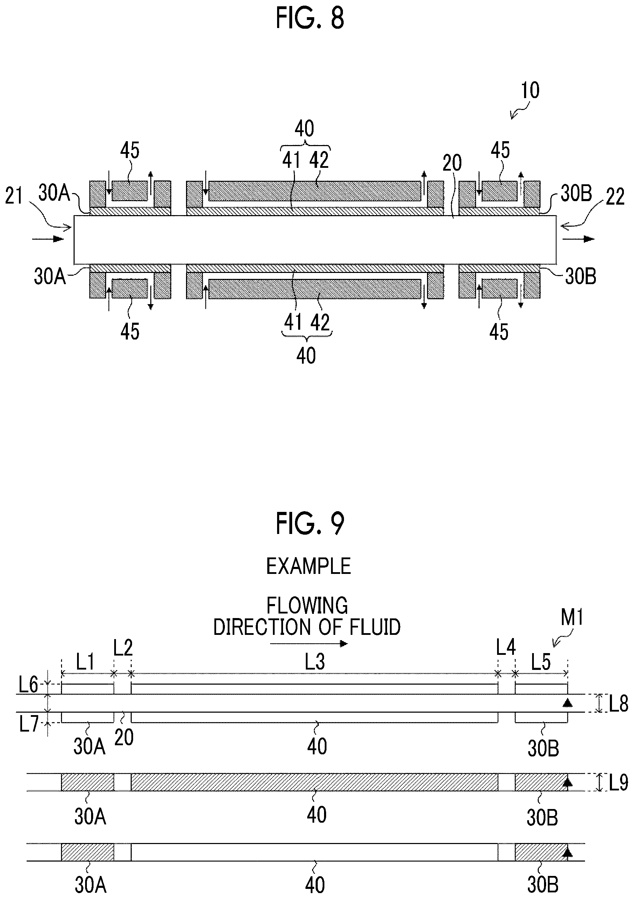

The electric perforating device 10 according to the embodiment of the disclosed technology can also be modified as shown in Fig. 8. That is, the electric perforating device 10 may include a cooling mechanism 45 for cooling the first electrode 30A and the second electrode 30B. Similar to the cooling mechanism 42 that constitutes the cooling portion 40, the cooling mechanism 45 may be a water cooling type mechanism through which liquid as a cooling medium flows inside the jacket. From the viewpoint of cooling efficiency, it is preferable that the coolant comes into direct contact with the first electrode 30A and the second electrode 30B. The cooling mechanism 45 is not limited to a water cooling type mechanism and may be, for example, an air cooling type mechanism or a mechanism using a Peltier element. In Fig. 8, although a configuration is shown in which the first electrode 30A, the second electrode 30B, and the heat conduction member 41 are cooled by separate cooling mechanisms, the cooling mechanism may be configured to integrally cool the first electrode 30A, the second electrode 30B, and the heat conduction member 41.

The local and instantaneous maximum temperature of the suspension flowing through the flow passage 20 rises during the ON period tₒₙ of the pulse voltage supplied to the first electrode 30A and the second electrode 30B and drops during the OFF period t_{off} of the pulse voltage. By cooling the first electrode 30A and the second electrode 30B, it is possible to increase the speed of decrease in the local and instantaneous maximum temperature of the suspension during the OFF period t_{off} of the pulse voltage. Accordingly, the local and instantaneous maximum temperature of the suspension can be suppressed. Further, since the temperatures of the first electrode 30A and the second electrode 30B are also suppressed, it is possible to suppress the progress of deterioration of these electrodes.

### [Computation Fluid Dynamics Analysis]

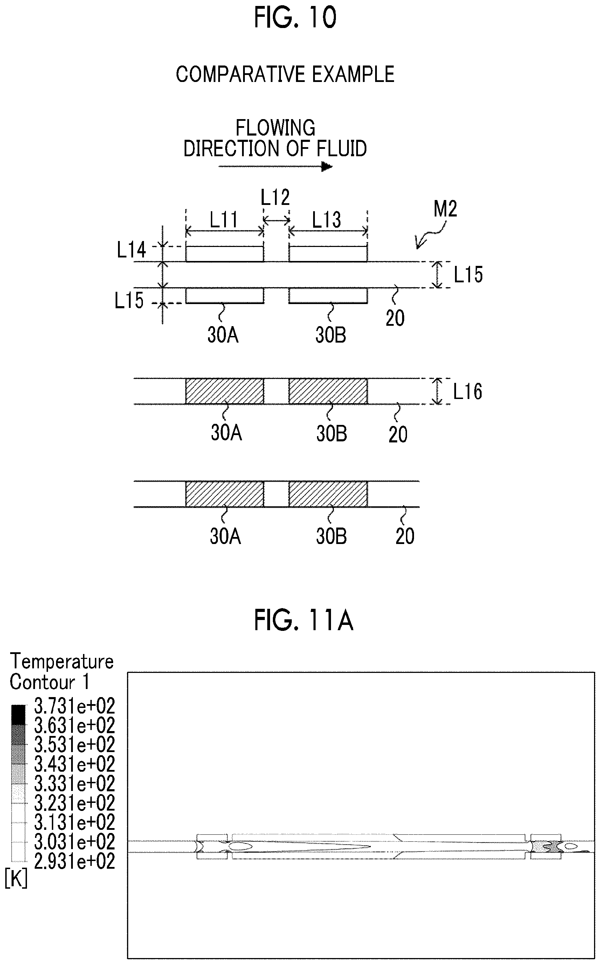

A computational fluid dynamics (CFD) analysis has been performed on an electric perforating device according to Example of the disclosed technology. Fig. 9 is a diagram showing a simulation model M1 of the electric perforating device according to Example of the disclosed technology. The upper part is a view showing a form seen from the side, and the middle part and the lower part are views showing forms seen from above. The model M1 according to Example of the disclosed technology includes the cooling portion 40 between the first electrode 30A and the second electrode 30B is a model for cooling the first electrode 30A and the second electrode 30B in a way equivalent to water cooling. Fig. 10 is a diagram showing a simulation model M2 of an electric perforating device according to Comparative Example of the disclosed technology. The upper part is a view showing a form seen from the side, and the middle part and the lower part are views showing forms seen from above. The model according to Comparative Example is a model for cooling the first electrode 30A and the second electrode 30B in a way equivalent to water cooling without including the cooling portion between the first electrode 30A and the second electrode 30B.

### (1) Dimensions of Flow Passage Model

In the model M1 according to Example of the disclosed technology shown in Fig. 9, the dimensions of each part are as follows.
L1 (a length of the first electrode 30A): 5 mm
L2 (an interval between the first electrode 30A and the cooling portion 40): 1 mm
L3 (a length of the cooling portion 40): 50 mm
L4 (an interval between the cooling portion 40 and the second electrode 30B): 1 mm
L5 (a length of the second electrode 30B): 5 mm
L6 (thicknesses of the first electrode 30A, the second electrode 30A, and the cooling portion 40): 1 mm
L7 (thicknesses of the first electrode 30A, the second electrode 30B, and the cooling portion 40): 1 mm
L8 (a height of the flow passage 20): 2 mm
L9 (a width of the flow passage 20): 2 mm

In the model M2 according to Comparative Example shown in Fig. 10, the dimensions of each part are as follows.
L11 (a length of the first electrode 30A): 5 mm
L12 (an interval between the first electrode 30A and the second electrode 30B): 1 mm
L13 (a length of the second electrode 30B): 5 mm
L14 (thicknesses of the first electrode 30A, the second electrode 30B, and the cooling portion 40): 1 mm
L15 (thicknesses of the first electrode 30A, the second electrode 30B, and the cooling portion 40): 1 mm
L16 (a width of the flow passage 20): 2 mm

### (2) Analysis Software (common to Examples and Comparative Examples)

### Ansys 2020R1

### (3) Mesh Condition (common to Examples and Comparative Examples)

Hexa-mesh standard cell size 0.2 mm
Mesh subdivision only on the wall surface of the flow passage (standard condition for an Ansys inflation function)

### (4) Analysis Model (common to Examples and Comparative Examples)

Viscous model: k-epsilon (standard condition)
Potential: Apply potential equation, incorporate Joule heating

### (5) Physical properties (common to Examples and Comparative Examples)

Physical properties of the fluid flowing through the flow passage 20, the first electrode 30A, the second electrode 30B, and the cooling portion 40 were set to values shown in Table 1 below. The physical properties of the fluid are equivalent to those of saline, and the physical properties of the first electrode 30A, the second electrode 30B, and the cooling portion 40 are equivalent to those of copper.

**[Table 1]**

| | Unit | Fluid | Electrode/ Cooling Portion |
|---|---|---|---|
| Density | kg/m³ | 1002 | 8960 |
| Specific Heat | j/kg·k | 4178 | 385 |
| Thermal Conductivity | w/m·k | 0.6 | 372 |
| Viscosity | kg/m·s | 1.00 × 10⁻³ | - |
| Conductivity | siemens/m | 1.6 | 5.95 × 10⁷ |

### (6) Boundary Condition

(a) Amount of Movement
   Inlet Velocity: 66.7 mm/s
(b) Heat
   Hatching portions in the middle part in Fig. 9 and the middle part in Fig. 10 Convection
   Convective Heat Transfer Coefficient: 7000 [W/m²·k]
   Free flow temperature: 283.15 [K] (equivalent to 10°C water cooling) Boundary portion where different materials are in contact
   Coupling
   All other wall surfaces
   Heat flux: 0 [W/m²] (adiabatic equivalent)
   Temperature of fluid inflowing from the inlet: 300 [k]
(c) Potential
   Hatching portions in the lower part in Fig. 9 and the lower part in Fig. 10 Pulse voltage application (see Fig. 3)
   Voltage: + 195 V and - 195 V are applied alternately
   Period T: 75 ms
   ON period tₒₙ: 5 ms
   OFF period t_{off}: 70 ms
   All other wall surfaces
   Current density: 0 [A/m²]

### (7) Calculation Condition

Non-stationary analysis
Time interval: 5ms

Regarding the convergence: After confirming the convergence of the model with a stationary analysis under the same condition, perform a non-stationary analysis.

### (8) Analysis Result

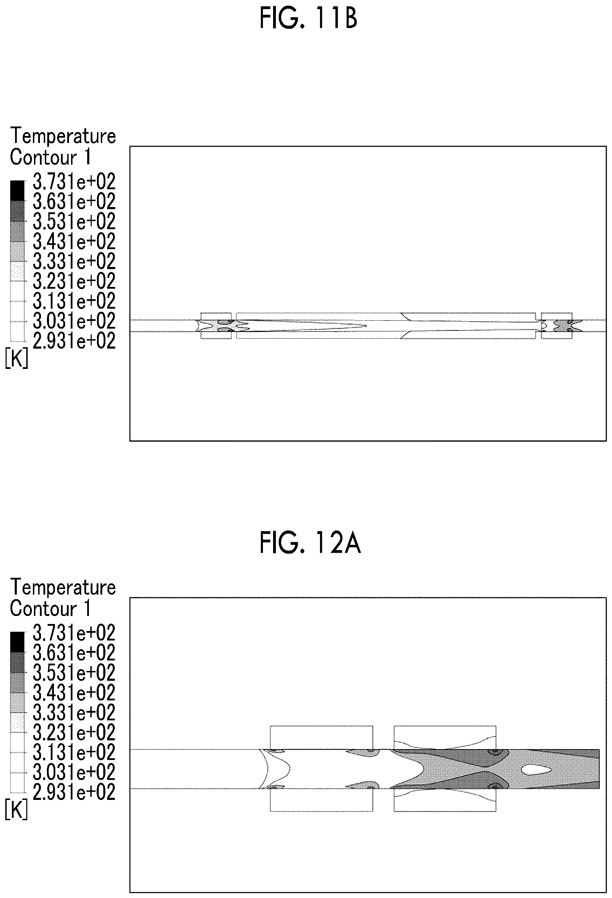

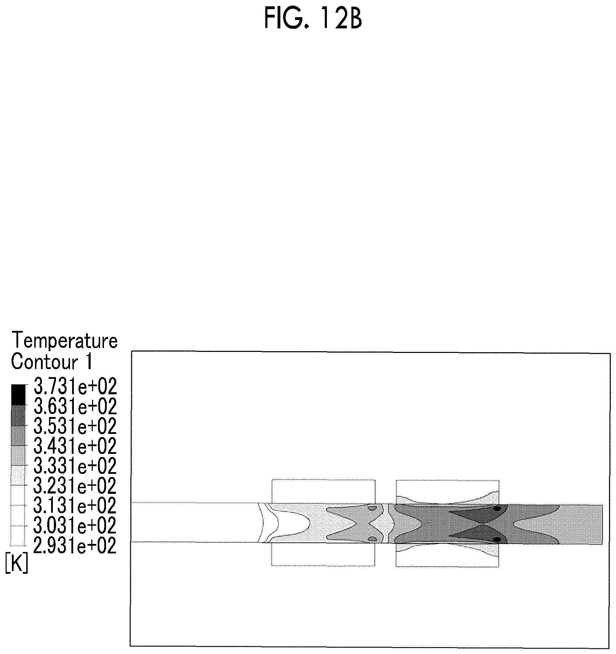

Figs. 11A and 11B are contour diagrams showing a temperature distribution of fluid in the model M1 according to Example of the disclosed technology. Fig. 11A is a temperature distribution in a cross section at a center of the flow passage 20 in the width direction, and Fig. 11B is a temperature distribution in a cross section in the vicinity of a wall surface of the flow passage 20. Figs. 12A and 12B are contour diagrams showing a temperature distribution of fluid in the model M2 according to Comparative Example. Fig. 12A is a temperature distribution in a cross section at the center of the flow passage 20 in the width direction, and Fig. 12B is a temperature distribution in a cross section in the vicinity of the wall surface of the flow passage 20. As shown in Figs. 11A and 11B, according to the model M1 of Example of the disclosed technology, fluid heated in the first electric field region flows into the second electric field region after being cooled in the cooling region. As shown in Figs. 12A and 12B, according to the model M2 of Comparative Example, the fluid heated in the first electric field region is further heated in the second electric field region without being cooled.

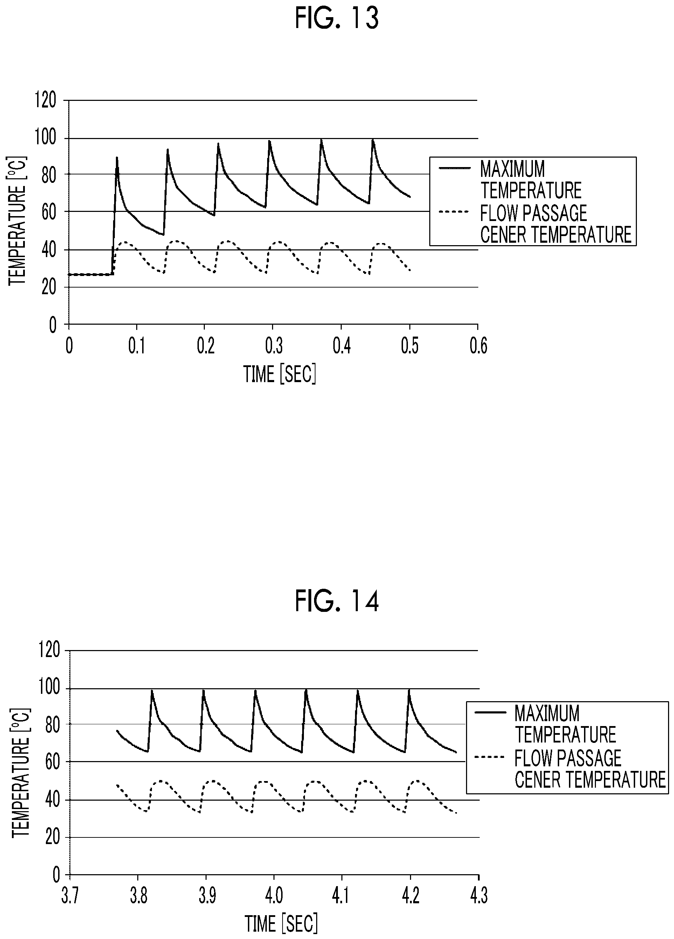

Figs. 13 and 14 are each graphs showing a time transition of the temperature of the fluid in the model M1 according to Example of the disclosed technology. Fig. 13 shows a time transition of the temperature of the fluid during a period immediately after the start of application of the pulse voltage, and Fig. 14 shows a time transition of the temperature of the fluid during a period before and after a timing of moment, four seconds after the start of application of the pulse voltage. In Figs. 13 and 14, the time transition of the maximum temperature of the fluid is indicated by a solid line, and the time transition of the temperature at the center of the flow passage 20 at an end terminal portion of the second electrode 30B in the width direction (a location marked with a triangle in Fig. 9) is indicated by a dotted line.

Table 2 below shows the presence or absence of a location where the temperature of the fluid is 100°C or higher for the model M1 according to Example and the model M2 according to Comparative Example of the disclosed technology. As shown in Table 2, in the model M1 according to Example of the disclosed technology, it was confirmed that there were no locations where the temperature of the fluid was 100°C or higher, that is, the maximum temperature of the fluid was 100°C or lower.

**[Table 2]**

| | Location where temperature of fluid is 100°C or higher |
|---|---|
| Example | No |
| Comparative Example | Yes |

### [Second Embodiment]

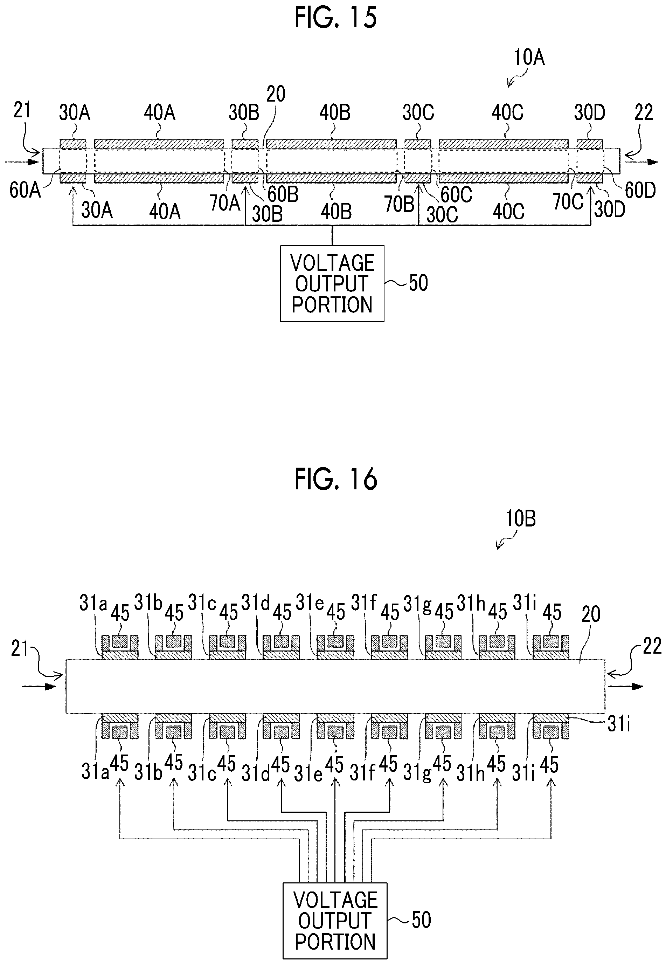

Fig. 15 is a cross-sectional view showing an example of a configuration of an electric perforating device 10A according to a second embodiment of the disclosed technology. The electric perforating device 10A according to the present embodiment includes a third electrode 30C and a fourth electrode 30D in addition to the first electrode 30A and the second electrode 30B. Further, the electric perforating device 10A according to the present embodiment includes a first cooling portion 40A, a second cooling portion 40B, and a third cooling portion 40C.

The third electrode 30C forms a third electric field region 60C on a downstream side of the second electric field region 60B, which is in the flow passage 20, in the flowing direction of the suspension by being supplied with a pulse voltage. The fourth electrode 30D forms a fourth electric field region 60D on a downstream side of the third electric field region 60C, which is in the flow passage 20, in the flowing direction of the suspension. The first cooling portion 40A forms a first cooling region 70A between the first electric field region 60A and the second electric field region 60B in the flow passage 20. The second cooling portion 40B forms a second cooling region 70B between the second electric field region 60B and the third electric field region 60C in the flow passage 20. The third cooling portion 40C forms a third cooling region 70C between the third electric field region 60C and the fourth electric field region 60D in the flow passage 20.

The voltage output portion 50 supplies a pulse voltage to each of the first electrode 30A, the second electrode 30B, the third electric field region 60C, and the fourth electrode 30D. The amount of energy E_{N} required for introducing the bioactive substance into the biologically derived material is divided and applied to the first to fourth electric field regions 60A to 60D. By making the dimensions of the first to fourth electrodes 30A to 30D the same (the length in the flowing direction of the suspension), the required amount of energy E_{N} can be divided evenly and applied to each electric field region.

According to the electric perforating device 10A of the present embodiment, similar to the first embodiment, an introduction method of introducing the bioactive substance into the biologically derived material while the suspension containing the biologically derived material and the bioactive substance flows through the flow passage, is implemented. The introduction method according to the present embodiment is a method in which a step of causing the suspension to pass through the electric field region and a step of causing the suspension to pass through the cooling region are alternately repeated two or more times each (in the example shown in Fig. 15, the step of causing the suspension to pass through the electric field region is performed four times, and the step of causing the suspension to pass through the cooling region is performed three times).

According to the electric perforating device 10A of the present embodiment, since more electric field regions can be formed than the electric perforating device 10 according to the first embodiment (see Figs. 1 and 2), the amount of energy in one electric field region can be further suppressed, and local and instantaneous temperature rises of the electrode and suspension can be further suppressed. The number of electrodes may be three or five or more. The cooling portion is provided between each electrode.

### [Third Embodiment]

Fig. 16 is a cross-sectional view showing an example of a configuration of an electric perforating device 10B according to a third embodiment of the disclosed technology. The electric perforating device 10B according to the present embodiment includes nine pairs of conductive members 31a to 31i provided on the wall surfaces of the flow passage 20 facing each other. The conductive members 31a to 31i are disposed along the flowing direction of the suspension flowing through the flow passage 20. Dimensions (a length in the flowing direction of the suspension) of the conductive members 31a to 31i are preferably the same. Further, the conductive members 31a to 31i are preferably made of a metal having relatively high conductivity and thermal conductivity, such as Cu and Al. The number of the conductive members is not limited and can be increased or decreased as appropriate.

The electric perforating device 10B includes the cooling mechanism 45 that individually cools each of the conductive members 31a to 31i. The cooling mechanism 45 may be, for example, a water cooling type mechanism through which liquid as a cooling medium flows inside a jacket, as illustrated in Fig. 16. From the viewpoint of cooling efficiency, it is preferable that coolant comes into direct contact with the conductive members 31a to 31i. Further, it is also possible to integrally configure the conductive members 31a to 31i and the cooling mechanism 45 by providing the flow passages through which the cooling medium flows inside the conductive members 31a to 31i. Further, the cooling mechanism 45 is not limited to a water cooling type mechanism and may be an air cooling type mechanism or a mechanism using a Peltier element. Although Fig. 16 illustrates a configuration in which the conductive members 31a to 31i are cooled by separate cooling mechanisms, the cooling mechanism may be configured to integrally cool the conductive members 31a to 31i.

The voltage output portion 50 can independently supply the pulse voltage to the conductive members 31a to 31i. The voltage output portion 50 supplies the pulse voltage to each of two pairs of conductive members between which at least one pair of conductive members is interposed. Accordingly, the conductive member, among the two pairs of conductive members supplied with the voltage pulse, on an upstream side in the flowing direction of the suspension functions as a first electrode that forms a first electric field region in the flow passage 20. Further, the conductive member, among the two pairs of conductive members supplied with the voltage pulse, on the downstream side in the flowing direction of the suspension functions as a second electrode that forms a second electric field region on the downstream side of the first electric field region, which is in the flow passage, in the flowing direction of the suspension. Further, at least one pair of conductive members, which is not supplied with the voltage pulse and which is interposed between the two pairs of conductive members supplied with the voltage pulse, functions as the cooling portion that forms the cooling region between the first electric field region and the second electric field region. That is, the conductive members 31a to 31i also function as heat conduction members that move the heat of the suspension to the cooling mechanism 45.

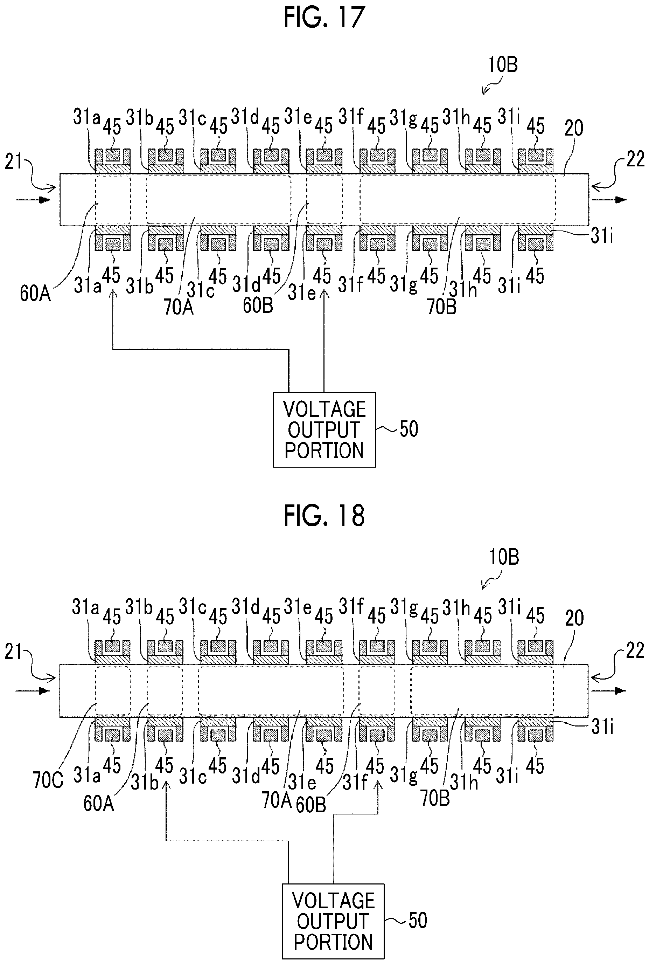

For example, as shown in Fig. 17, the voltage output portion 50 supplies the pulse voltage to each of the conductive members 31a and 31e. Accordingly, the conductive member 31a functions as the first electrode that forms the first electric field region 60A in the flow passage, and the conductive member 31e functions as the second electrode that forms the second electric field region 60B on the downstream side of the first electric field region 60A, which is in the flow passage, in the flowing direction of the suspension. The conductive members 31b, 31c, and 31d, which are not supplied with the voltage pulse and which are interposed between the conductive member 31a and the conductive member 31e, function as a first cooling portion that forms the first cooling region 70A between the first electric field region 60A and the second electric field region 60B in the flow passage 20. Further, the conductive members 31f, 31g, 31h, and 31i, which are not supplied with the voltage pulse, function as a second cooling portion that forms the second cooling region 70B on the downstream side of the second electric field region 60B, which is in the flow passage 20, in the flowing direction of the suspension.

The voltage output portion 50 switches the conductive member, to which the pulse voltage is supplied among the plurality of pairs of conductive members, at a predetermined timing. As shown in Fig. 18, for example, the voltage output portion 50 switches the conductive members 31b and 31f as the conductive members to which the pulse voltage is supplied. Accordingly, the conductive member 31b functions as the first electrode that forms the first electric field region 60A in the flow passage, and the conductive member 31f functions as the second electrode that forms the second electric field region 60B on the downstream side of the first electric field region 60A, which is in the flow passage, in the flowing direction of the suspension. The conductive members 31c, 31d, and 31e, which are not supplied with the voltage pulse and which are interposed between the conductive member 31b and the conductive member 31f, function as a first cooling portion that forms the first cooling region 70A between the first electric field region 60A and the second electric field region 60B in the flow passage 20. Further, the conductive members 31g, 31h, and 31i, which are not supplied with the voltage pulse, function as the second cooling portion that forms the second cooling region 70B on the downstream side of the second electric field region 60B, which is in the flow passage, in the flowing direction of the suspension. Further, the conductive member 31a, which is not supplied with the voltage pulse, functions as a third cooling portion that forms the third cooling region 70C on an upstream side of the first electric field region 60A, which is in the flow passage, in the flowing direction of the suspension.

The voltage output portion 50 may switch the conductive member to which the pulse voltage is supplied, for example, at a timing when cumulative time from the start of the operation of the electric perforating device 10B exceeds a predetermined threshold value. Further, the voltage output portion 50 may switch the conductive member to which the pulse voltage is supplied at a timing when an amount of cumulative processing on the suspension in the electric perforating device 10B exceeds the predetermined threshold value. Further, the voltage output portion 50 may switch the conductive member to which the pulse voltage is supplied at a timing when the number of pulses of the pulse voltage output by the voltage output portion 50 exceeds the predetermined threshold value. Further, the voltage output portion 50 may switch the conductive member to which the pulse voltage is supplied at a timing when an abnormality is detected. The abnormality may be, for example, an abnormality in a current value, a resistance value, or a metal density value acquired in an in-line measurement. Further, the voltage output portion 50 may switch the conductive members to which the pulse voltage is supplied in a manner in which two pairs of the conductive members to which the pulse voltage is supplied are sequentially shifted along the flowing direction of the suspension.

In Figs. 16 and 17, although an example in which three conductive members adjacent to each other are used as conductive members that form a cooling region between the first electric field region 60A and the second electric field region 60B has been illustrated, the number of conductive members that form a cooling region between the first electric field region 60A and the second electric field region 60B can be increased or decreased as appropriate.

According to the electric perforating device 10B of the present embodiment, it is possible to suppress the local and instantaneous temperature rises of the electrode and the suspension as in the electric perforating device 10 of the first embodiment. Further, according to the electric perforating device 10B of the present embodiment, it is possible to switch the electrode that forms an electric field region by switching the conductive member to which the pulse voltage is supplied. Accordingly, even in a case where the conductive member previously used as the electrode deteriorates and the function as an electrode is decreased, since another conductive member that has not deteriorated can function as an electrode, the replacement frequency of the electric perforating device can be reduced. The replacement of the electric perforating device is performed in accordance with the loss of the suspension. Further, the liquid flow of the suspension becomes unstable before and after the replacement of the electric perforating device. Therefore, it is preferable that the replacement frequency of the electric perforating device is low. Further, by making the dimensions (the lengths in the flowing direction of the suspension) of the conductive members 31a to 31i the same, the occurrence of a change in the environment inside the flow passage 20 can be suppressed before and after switching the conductive member to which the pulse voltage is supplied.

In addition, the disclosure of JP2021-072675 filed on April 22, 2021 is incorporated herein by reference in its entirety. Further, all documents, patent applications, and technical standards described in the specification are incorporated herein by references to the same extent as the incorporation of the individual documents, patent applications, and technical standards by references are described specifically and individually.

## Claims

1. An electric perforating device comprising:
a flow passage through which liquid flows;
a first electrode that forms a first electric field region in the flow passage by being supplied with a pulse voltage;
a second electrode that forms a second electric field region on a downstream side of the first electric field region, which is in the flow passage, in a flowing direction of the liquid, by being supplied with a pulse voltage;
a cooling portion that forms a cooling region, which cools the liquid flowing through the flow passage, between the first electric field region and the second electric field region in the flow passage; and
a voltage output portion that outputs a pulse voltage for forming the first electric field region and the second electric field region.

2. The electric perforating device according to claim 1, further comprising:
a cooling mechanism that cools the first electrode and the second electrode.

3. The electric perforating device according to claim 1 or 2, further comprising:
a plurality of pairs of conductive members provided on wall surfaces of the flow passage facing each other along the flowing direction of the liquid; and
a cooling mechanism that cools each of the plurality of pairs of conductive members,
wherein, by supplying a pulse voltage with the voltage output portion to each of two pairs of conductive members between which at least one pair of conductive members, among the plurality of pairs of conductive members, is interposed, one of the two pairs of conductive members functions as the first electrode, the other of the two pairs of conductive members functions as the second electrode, and the at least one pair of conductive members, which is interposed between the two pairs of conductive members, functions as the cooling portion.

4. The electric perforating device according to claim 3,
wherein the voltage output portion switches the conductive member, to which the pulse voltage is supplied among the plurality of pairs of conductive members, at a predetermined timing.

5. The electric perforating device according to claim 3 or 4,
wherein the plurality of pairs of conductive members have the same length in the flowing direction of the liquid.

6. An introduction method of introducing a bioactive substance into a biologically derived material while suspension containing the biologically derived material and the bioactive substance flows through a flow passage, the introduction method comprising:
a step of causing the suspension to pass through a first electric field region;
a step of causing the suspension to pass through a cooling region, which cools the suspension, after the suspension has passed through the first electric field region; and
a step of causing the suspension to pass through a second electric field region after the suspension has passed through the cooling region.

7. The introduction method according to claim 6,
wherein a step of causing the suspension to pass through an electric field region and the step of causing the suspension to pass through the cooling region are alternately repeated two or more times each.

8. The introduction method according to claim 6 or 7,
wherein an accumulated amount of energy, which is applied to the suspension while the suspension passes through a plurality of electric field regions including the first electric field region and the second electric field region, corresponds to an amount of energy predetermined as energy required for introducing the bioactive substance into the biologically derived material.

9. The introduction method according to any one of claims 6 to 8,
wherein a local and instantaneous maximum temperature of the suspension in the flow passage is 100°C or lower.

10. The introduction method according to any one of claims 6 to 9,
wherein a plurality of electric field regions including the first electric field region and the second electric field region are each formed by supplying a pulse voltage to a cooled electrode.

11. The introduction method according to any one of claims 6 to 10,
wherein a plurality of electric field regions including the first electric field region and the second electric field region are each formed by supplying a pulse voltage to an electrode, and
time required for the suspension to pass through one electric field region is an integer multiple of a period of the pulse voltage.

12. The introduction method according to any one of claims 6 to 11,
wherein the biologically derived material is a human derived cell, and
the bioactive substance is DNA.

13. A manufacturing method of a biologically derived material comprising a step of introducing a bioactive substance by using the introduction method according to any one of claims 6 to 12.

14. A biologically derived material manufactured by using the manufacturing method according to claim 13.
